# EUROPÄISCHE PATENTANMELDUNG

(11) **EP 2 045 235 A1**
(43) Veröffentlichungstag der Anmeldung: **08.04.2009**
(21) Anmeldenummer: 07019318.0
(22) Anmeldetag: 02.10.2007
(51) Int. Cl.: C07C 227/08, C07C 227/18, C07C 229/16

(54) **Verfahren zur Herstellung von (S)-(+)-1,2-Diaminopropan-N,N,N',N'-tetraessigsäuretetraestern**

(71) Anmelder: Loba Feinchemie AG, 2401 Fischamend (AT)
(72) Erfinder: Marchalin, Miroslav, dr., 2401 Fischamend (AT)
(74) Vertreter: Landgraf, Elvira

(57) **Zusammenfassung**

Die Erfindung betrifft ein verbessertes Verfahren zur Herstellung von 1,2-Diaminopropan-tetraessigsäuretetraestern durch Alkylierung von 1,2-Diaminopropan Dihydrochlorid und anschließende Veresterung, dadurch gekennzeichnet, dass 1,2-Diaminopropan Dihydrochlorid mit Chloressigsäure unter Kühlung bei einer Temperatur von 0-15°C in Verbindung gebracht wird, worauf NaOH unter Kühlung bei einer Temperatur von 0 - 15°C zugegeben wird, wobei das molare Verhältnis 1,2-Diaminopropan Dihydrochlorid:Chloressigsäure:NaOH = 1:6:12 beträgt, worauf die Alkylierung bei einer Temperatur von 20 - 25°C erfolgt und im Anschluss gegebenenfalls weitere 2 MolÄ NaOH zur vollständigen Umsatzung zugegeben, worauf das Reaktionsgemisch unter Kühlung auf 10 - 17 °C durch Zugabe einer Säure auf einen pH-Wert von 7 - 8 eingestellt wird,

## Beschreibung

Die Erfindung betrifft ein verbessertes Verfahren zur Herstellung von (S)-(+)-1,2-Diaminopropan-N,N,N',N'-tetraessigsäuretetraestern, insbesondere (S)-(+)-1,2-Diaminopropan-N,N,N',N'-tetraessigsäuretetramethylester.

(S)-(+)-1,2-Diaminopropan-N,N,N',N'-tetraessigsäuretetramethylester ist ein Zwischenprodukt bei der Herstellung von (S)-(+)-4,4'-(1-Methyl-1,2-ethandiyl)bis-(2,6-piperazidon) (Dexrazoxan).

Dem Verfahren liegt zugrunde, dass (S)-(-)-1,2-Diaminopropan Dihydrochlorid im alkalischen Milieu mit Chloressigsäure, respektive deren Natriumsalz zur Tetraessigsäure alkyliert, diese ohne Isolierung der Alkylierungsprodukte verestert wird und erst der Ester (wegen der Löslichkeit des Esters sind Methyl-und Ethylester vorzuziehen) einem Reinigungs- und Isolierungsschritt unterworfen wird.

Für die Reinheit des Endproduktes ist eine hohe Enantiomerenreinheit des Ausgangsproduktes (S)-(-)-1,2-Diaminopropan Dihydrochlorid entscheidend. Um die Wirtschaftlichkeit des Verfahrens zu gewährleisten wird ein möglichst hoher Reaktionsumsatz angestrebt.

Im erfindungsgemäßen Verfahren wird durch eine auf die Reaktionsbedingungen hin verbesserte Führung des Verfahrens bei der Alkylierung, der Veresterung und der Reinigung eine deutliche Steigerung der Ausbeute, der Prozesssicherheit (Reproduzierbarkeit!) und damit Wirtschaftlichkeit des Verfahrens erzielt. (S)-(+)-1,2-Diaminopropan-N,N,N',N'-tetraessigsäuretetramethylester

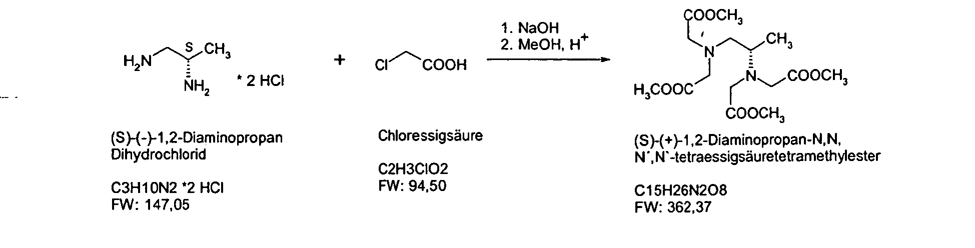

Um die Hydrolyse der Chloressigsäure so gering wie möglich zu halten erfolgt die Vereinigung der Reaktionspartner bei der Alkylierung unter Kühlung - der optimale Temperaturbereich beträgt dabei 0-15°C,

Das molare Verhältnis der Reaktionspartner beträgt vorzugsweise (S)-(-)-1,2-Diaminopropan Dihydrochlorid : Chloressigsäure : Natriumhydroxid = 1: 6 : 12

Das Natriumhydroxid wird unter Kühlung zugefügt, um die Hydrolyse der Chloressigsäure so gering wie möglich zu halten, der optimaler Temperaturbereich beträgt dabei 0-15°C,

Die Reaktion der Alkylierung erfolgt bei 20-25°C (Raumtemperatur) über 145-190 Std (6-8 Tage), optimal 170 Std (7 Tage);

Um etwaige Reste an Chloressigsäure noch zur Reaktion zu bringen (z.B. mit (S)-(-)-1,2-Diaminopropan Di HCl) werden weitere 2 molÄ Natriumhydroxid zugesetzt und das Reaktionsgemisch kurz (30 min) auf 70-80°C erhitzt.

Bei der Veresterung erfolgt die Neutralisation der stark basischen Alkylierungs-Reaktionslösung vor der Zugabe des Alkohols für die Veresterung;

Die Neutralisation wird mit dem isolierten Reaktionsgemisch ein weiteres Mal durchgeführt;

Die weitere Veresterung erfolgt nach einer Neutralisation, abtrennen der Salzfracht vom Reaktionsgemisch;

Die Reinigung des Tetrametylesters von unerwünschten Nebenprodukten erfolgt durch verteilen der Reaktionsprodukte zwischen einer stark sauren wässrigen und einer organischen Phase.

Zur Abtrennung von weiteren Nebenprodukten und der vornehmlichen Isolierung des Tetramethylesters wird die wässrige Extraktionsphase auf neutral gestellt (pH 7) und mit einem organischen Lösungsmittel das Produkt extrahiert, welches nach abtrennen des Lösungsmittels als nahezu farbloses Öl gewonnen wird.

Dadurch wird das Endprodukt (S)-(+)-1,2-Diaminopropan-N,N,N',N'-tetraessigsäuretetraester in höherer Ausbeute, und Reinheit und mit höherer Reproduzierbarkeit erhalten und kann zur Herstellung von (S)-(+)-4,4'-(1-Methyl-1,2-ethandiyl)bis-(2,6-piperazidon) (Dexrazoxan) verwendet werden.

### Beispiele

### Beispiel 1: (Stand der Tecknik)

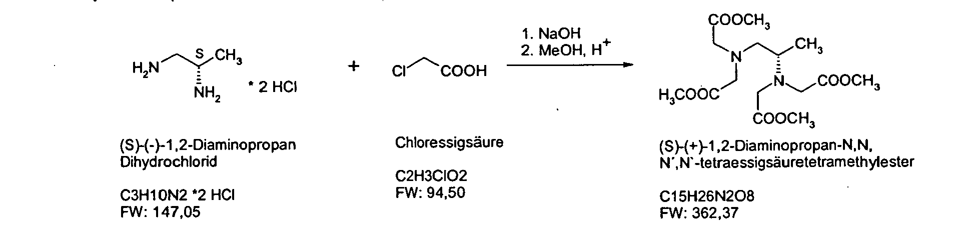

In 1170 ml Wasser deionisiert werden 225 g (1,53 mol) 1,2-Diaminopropan DiHCl und 867,6 g (9,18 mol) Chloressigsäure (6,0 molÄ) gelöst, wobei es durch eine endotherme Reaktion zu einer Temperaturerniedrigung von 22 auf 6°C kommt - es wird so lange gerührt, bis sich alles in Lösung befindet. Die Lösung wird auf 0°C gekühlt und unter Kühlung bei T <15°C 2677,5 g (21,42 mol) Natronlauge 32% (14 molÄ) über 1 Std. zudosiert.

Nach vollständiger Zugabe der Natronlauge wird die Lösung erwärmt - innerhalb 1 Std auf 45°C und bei 45-50°C über 97 Std gerührt, wobei sich ein End-pH 13-14 einstellt.

Unter Vak (70-80 mbar) bei einer Badtemp. von 50-70°C wird das Gemisch bis z.Tr. eingeengt - 2763 g Destillat, 2071 g Rückstand. Dieser wird mit 1800 ml MeOH aufgenommen, gerührt, abgesaugt, der Filter mit 2x450 ml MeOH nachgewaschen und das Filtrat unter Vak bis z.Tr. eingeengt - 1235,6 g Rückstand.

Zur Veresterung wird der Rückstand mit 4,5 Ltr methanolischer Schwefelsäure (4050 ml MeOH + 405 ml Schwefelsäure 95%ig) versetzt und zum Sieden erhitzt. Nach 3 Std werden 11,3 ml Schwefelsäure und nach 3 weiteren Std nochmals 11,3 ml zugesetzt. Insgesamt wird 10 Std refluxiert.

Nach erkalten der Lösung wird mit 450 g Natriumbicarbonat neutralisiert, 2 Std nachgerührt, filtriert, der Salz-Rückstand mit 675 ml MeOH nachgewaschen und das Filtrat unter Vak bis z.Tr. eingeengt . Der Rückstand wird mit 1125 ml Essigsäureethylester und 1275 ml 2N Salzsäure versetzt und extrahiert. Nach der Phasentrennung wird die organische Phase abgetrennt und verworfen. Die wässrige Phase wird mit 1x 1125 ml Essigsäureethylester nochmals extrahiert, welche ebenfalls verworfen wird. Die wässrige Phase wird mit 800 ml Essigsäureethylester überschichtet und mit 300 g Natriumbicarbonat neutralisiert, mit weiteren 888 ml Essigester versetzt, extrahiert. Die abgetrennte wässrige Phase wird abermals mit 2x je 1125 ml Essigester extrahiert, die abgetrennte wässrige Phase verworfen, die vereinigten organischen Phasen unter Vakuum bis zur Trockene eingeengt. Ausbeute: 297,7 g Produkt (53,7 % dTh; Th: 554.47 g; Soll: 55-70 %dTh (305-388 g)) Analytik: HPLC : 1,2-Diaminopropan-tetraessigsäuretetramethylester: 53,94 Fl%, Nebenprodukt 1,2-Diaminopropan-triessigsäuretrimethylester: 43,28 Fl%

### Beispiel 2: (erfindungsgemäßes Verfahren)

In 520 g Wasser deionisiert werden 100 g (0,680 mol) 1,2-Diaminopropan DiHCl und 385,6 g (4,08 mol) Chloressigsäure (6 molÄ), wobei es durch eine endotherme Reaktion zu einer Temperaturemiedrigung auf 5°C kommt, gelöst. Es wird so lange gerührt, bis sich alles in Lösung befindet. Die klare Lösung wird auf 0°C gekühlt und unter Kühlung bei T <15°C 1020,0 g (8,42 mol) Natronlauge 32% (12,37 molÄ) über 1,5 Std. zudosiert - bis zur Hälfte der Zugabe stark exotherm, dann keine Wärmetönung feststellbar.
Nach vollständiger Zugabe der Natronlauge wird die Lösung bei RT (21-24°C) über 168 Std (7Tage) gerührt, wobei der pH von 14,3 auf 12,8 sinkt.
Nachhydrolyse: Dem Reaktionsgemisch werden 170,0 g g Natronlauge 32% zugesetzt und für 30 min auf 80 °C erhitzt.
Das Reaktionsgemisch wird auf 15°C abgekühlt und mit 114,8g Salzsäure konz innerhalb von 20 min unter Kühlung auf pH 7,6 eingestellt.

Unter Vakuum wird das Gemisch bis zur Trockenen eingeengt (Rückstand 899 g), mit 800 ml Methanol versetzt, 15 min bei 60°C gerührt, auf 20°C abgekühlt, die unlöslichen Salze über eine Nutsche abgesaugt, 2x mit je 200 ml Methanol nachgewaschen und das Filtrat unter Vakuum bis zur Trockenen eingeengt - Rückstand 544 g.

Zur Veresterung wird der Rückstand, 544 g ölige Masse, wird mit 300 ml Methanol bei 60°C über 30 min gerührt, mit 1500 ml Methanol versetzt, auf 18°C gekühlt und das Gemisch mit 282,8 g (2,739 mol) Schwefelsäure konz versetzt, wobei die Temperatur auf 53°C steigt. Das Reaktionsgemisch wird unter rühren über 12 Std auf 58-60°C erhitzt. Nach 3 und 6 Std werden jeweils 5,0 ml Schwefelsäure konz nachgesetzt.

Das Reaktionsgemisch wird abgekühlt und portionsweise mit 176 g (2,1 mol) Natriumhydrogencarbonat neutralisiert, nach vollständiger Zugabe 2 Std bei RT weitergerührt, die anorganischen Salze abgesaugt, mit 2x je 155 ml Methanol nachgewaschen und das Filtrat unter Vakuum zur Trockenen eingeengt - Rückstand 468 g.

Der Rückstand, 468 g öliger Kristallbrei, wird mit 1850 ml Methanol und 111,6 g Schwefelsäure konz versetzt (ph 1-1,5) und unter rühren 8 Std auf 58-60°C erhitzt. Das abgekühlte Reaktionsgemisch wird portionsweise mit 88 g (1,05 mol) Natriumhydrogencarbonat neutralisiert, nach vollständiger Zugabe 2 Std bei RT weitergerührt, die unlöslichen Salze abgesaugt, der Salz-Rückstand mit 2x 155 ml Methanol gewaschen und das Filtrat unter Vakuum bis zur Trockenen eingeengt - 560,8 g öliger Rückstand.

Dieser wird zwischen 240 ml Wasser deionisiert und 515 ml Essigsäureethylester verteilt, bei 15°C mit 298,8 g 11,5 %iger Salzsäure versetzt und 5 min gerührt. Die organische Phase wird abgetrennt und die wässrige Phase mit 514 und 257 ml Essigsäureethylester extrahiert - die organischen Phasen werden verworfen.

Die wässrige Phase wird mit 514 ml Essigsäureethylester überschichtet und mit 96 g Natriumhydrogencarbonat innerhalb 1 Std der pH auf 7 gestellt. Mit weiteren 257 ml Essigsäureethylester wird extrahiert, die organische Phase abgetrennt und nochmals 2x mit je 514 ml Essigsäureethylester extrahiert. Die vereinigten organischen Phasen werden über Natriumsulfat getrocknet und unter Vakuum bis zur Trockenen eigeengt. Anschließend wird noch bei 60°C 30 min entgast. Ausbeute 155,9 g nahezu farbloses, dünnflüssiges Öl (63,3 % dTh; Th: 246,43g) Analytik: HPLC : 1,2-Diaminopropan-tetraessigsäuretetramethylester: 93,26 Fl%, Nebenprodukt 1,2-Diaminopropan-triessigsäuretrimethylester: 4,36 Fl%

Damit konnte die Ausbeute verdoppelt werden. In Anbetracht der hohen Rohstoffpreise, der nicht unwesentlichen Produktionskosten eine bedeutende Steigerung der Wertschöpfung.

Beispiel 3 - Verwendung zur Herstellung von Dexrazoxan (S)-(+)-4,4'-(1-Methyl-1,2-ethandiyl)bis-(2,6-piperazidon); INN "Dexrazoxan" 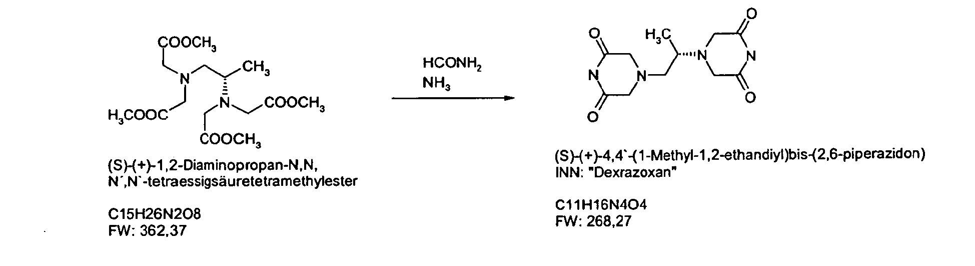 Die Herstellung kann gemäß dem bekannten Stand der Technik erfolgen.

Die Einleitung des gasförmigen Ammoniaks erfolgt drucklos, da besonders durch Kühlung des Reaktionsgemisches die Löslichkeit des Gases verbessert wird; bei der Reinigung des Rohproduktes ein 2-faches umkristallisieren aus Dioxan bei möglichst niedriger Temperatur und anschließendem umkristallisieren aus Wasser einen ausreichenden Reinigungseffekt liefert.
Wesentlich ist auch, in allen Verfahrensschritten darauf zu achten, dass ab der Alkylierung der sehr gute Chelator mit keinen mehrwertigen Metallionen in Berührung kommt (besonders bei der Wasserqualität darauf zu achten).

## Patentansprüche

1. Verbessertes Verfahren zur Herstellung von 1,2-Diaminopropantetraessigsäuretetraestern durch Alkylierung von 1,2-Diaminopropan Dihydrochlorid und anschließende Veresterung, **dadurch gekennzeichnet, dass** 1,2-Diaminopropan Dihydrochlorid mit Chloressigsäure unter Kühlung bei einer Temperatur von 0-15°C in Verbindung gebracht wird, worauf NaOH unter Kühlung bei einer Temperatur von 0 - 15°C zugegeben wird, wobei das molare Verhältnis 1,2-Diaminopropan Dihydrochlorid:Chloressigsäure:NaOH = 1:6:12 beträgt, worauf die Alkylierung bei einer Temperatur von 20 - 25°C erfolgt und im Anschluss gegebenenfalls weitere 2 MolÄ NaOH zur vollständigen Umsatzung zugegeben, worauf das Reaktionsgemisch unter Kühlung auf 10 - 17 °C durch Zugabe einer Säure auf einen pH-Wert von 7 - 8 eingestellt wird, anschließend zur Veresterung ein Alkohol zugegeben wird, worauf wiederum mit einer Säure neutralisiert wird und die Reinigung des Esters durch Verteilen der Reaktionsprodukte zwischen einer stark sauren wässrigen und einer organischen Extraktionsphase erfolgt.

2. Verfahren nach Anspruch 1, **dadurch gekennzeichnet, dass** zur Isolierung des Tetraesters die wässrige Extraktionsphase anschließend auf pH 7 eingestellt und mit einem organischen Lösungsmittel extrahiert wird.

3. Verfahren nach einem der Ansprüche 1 bis 2, **dadurch gekennzeichnet, dass** als Säure Salzsäure verwendet wird.

4. Verfahren nach einem der Ansprüche 1 bis 3, **dadurch gekennzeichnet, dass** als Alkohol Methanol oder Ethanol verwendet wird.
